# EUROPEAN PATENT APPLICATION

(11) **EP 1 808 190 A1**
(43) Date of publication of application: **18.07.2007**
(21) Application number: 07100245.5
(22) Date of filing: 08.01.2007
(51) Int. Cl.: A61M 1/28

(54) **Dialysis Equipment Bag**

(30) Priority: 11.01.2006 GB 0600463
(71) Applicant: McCarthy, John, Huddersfield, Yorkshire HD2 1PZ (GB)
(72) Inventor: McCarthy, John, Huddersfield, Yorkshire HD2 1PZ (GB)
(74) Representative: Kohler, Janet Wendy

(57) **Abstract**

A dialysis equipment bag (10), wherein the dialysis equipment bag (10) is adapted to receive at least one dialysis bag (18); and the dialysis equipment bag (10) comprises heating means (36,38) for warming said at least one dialysis bag (18).

## Description

### FIELD OF THE INVENTION

The present invention relates to a dialysis equipment bag for carrying and warming one or more dialysis bags and a method of using such a dialysis equipment bag.

### BACKGROUND TO THE INVENTION

Many patients suffering from end stage renal failure opt to start dialysis using continuous ambulatory peritoneal dialysis (CAPD). This method of dialysis offers a greater level of independence than attending hospital every two days. Furthermore, this method gives them a greater degree of control over their own health. CAPD is designed to be performed anywhere, thus fitting in around the patients lifestyle. However, in practice this is not always possible, given the high degree of sterilisation required to perform CAPD, the amount of associated material needed, and the ability to warm the bags.

In order to perform CAPD dialysis bags are provided. These bags contain a fresh dialysis solution bag, an empty waste products bag and tubing for connecting the bag to the body of the patient. The dialysis solution is warmed to body temperature prior to use, for comfort when it enters the body of the patient.

Current apparatus for warming dialysis bags is designed for use in the home. It is heavy and awkward to carry and therefore not suitable for use when the patient wishes to travel.

It is an aim of the present invention to overcome at least one problem associated with the prior art whether referred to herein or otherwise.

In particular, an aim of the present invention is to enable a person requiring to perform CAPD to travel freely whilst still being able to perform CAPD.

### SUMMARY OF THE INVENTION

According to a first aspect of the present invention there is provided a dialysis equipment bag, wherein the dialysis equipment bag is adapted to receive at least one dialysis bag; and the dialysis equipment bag comprises heating means for warming said at least one dialysis bag.

Preferably, the dialysis equipment bag comprises a dialysis bag compartment adapted to receive the or each dialysis bag. Preferably, the heating means are located in the dialysis bag compartment.

Preferably the heating means are located in at least one wall or floor of the dialysis bag compartment. The heating means are preferably located in a pocket.

Preferably the heating means are electrical heating means. The heating means may be powered by the mains and/or operated by a car battery.

Preferably the heating means comprises at least one heating element. Preferably the at least one heating element comprises a first heating element which is connectable to the mains and a second heating element which is connectable to the car battery. Preferably both first and second heating elements provide a 50% coverage of the heated surface area of the at least one dialysis bag.

Preferably the at least one heating element is embedded in a flexible rubber mat, which may be made of silicone rubber. Preferably the at least one heating element is an etch foil element with a layer of rubber each side. The at least one heating element may be provided with a bimetal thermostat. The thermostat is preferably adapted to maintain a temperature at or around body temperature. Preferably the thermostat cuts out at 50°C and resets at 35°C.

Preferably padding is provided each side of the matting to protect the mat.

Preferably the dialysis bag compartment is sized such that the or each dialysis bag fits within the compartment without substantial play.

Preferably the dialysis equipment bag is provided with one or more additional storage compartments for carrying objects other than dialysis bags.

Preferably the dialysis equipment bag is made of lightweight material and/or waterproof material.

Preferably the dialysis equipment bag is designed to accommodate at least one power supply connection means. The or each power supply connection means may comprise a cable and a plug. The or each power supply connection means may be accommodated within the dialysis bag compartment or may be held in a separate location within the bag. The or each cable may pass through a hole in the wall of the compartment. The cable may be permanently connected to the heating elements.

Preferably the or each dialysis bag compartment is lined with heat storing material. The heat storing material is preferably able to maintain heat for six to eight hours.

Preferably foil is provided behind the or each heating means to reflect heat back into the dialysis bag compartment. Silver backing may be provided on the inside of the dialysis bag compartment to help maintain heat within the dialysis bag compartment.

A light signal or audible alarm may be provided externally or internally of the dialysis equipment bag to warn the user that the dialysis bags are at a usable temperature. The light signal may be provided by coloured LEDs or heat sensitive strips that change colour with temperature.

Preferably the dialysis equipment bag is a rucksack or a briefcase. The briefcase may be provided in two parts, the first part being adapted for use as a conventional briefcase, and the second part being adapted to carry and heat the dialysis bags.

The dialysis equipment bag is preferably provided with a card slot for receiving a card which informs the person reading it that the bag contains dialysis equipment.

According to a second aspect of the invention there is provided a method of operating the dialysis equipment bag when the dialysis equipment bag is as herein referred to.

The present invention includes any combination of the herein referred to features or limitations.

### BRIEF DESCRIPTION OF THE DRAWINGS

The present invention will now be described by way of example only with reference to the drawings that follow, in which:
Figure 1 is a front view of a bag according to a first embodiment of the present invention;
Figure 2 is a rear view of the bag of Figure 1;
Figure 3 is a side view of the bag of Figure 1;
Figure 4 is a schematic view of the inside of the bag of Figure 1;
Figure 5 is a perspective view showing a bag according to a second embodiment of the present invention;
Figure 6 is a perspective view showing the two-part form of the bag of Figure 5;
Figure 7 is a cross-sectional view of the bag of Figure 5; and
Figure 8 is a plan view of a heating arrangement suitable for use in the present invention.

### DESCRIPTION OF THE PREFERRED EMBODIMENTS

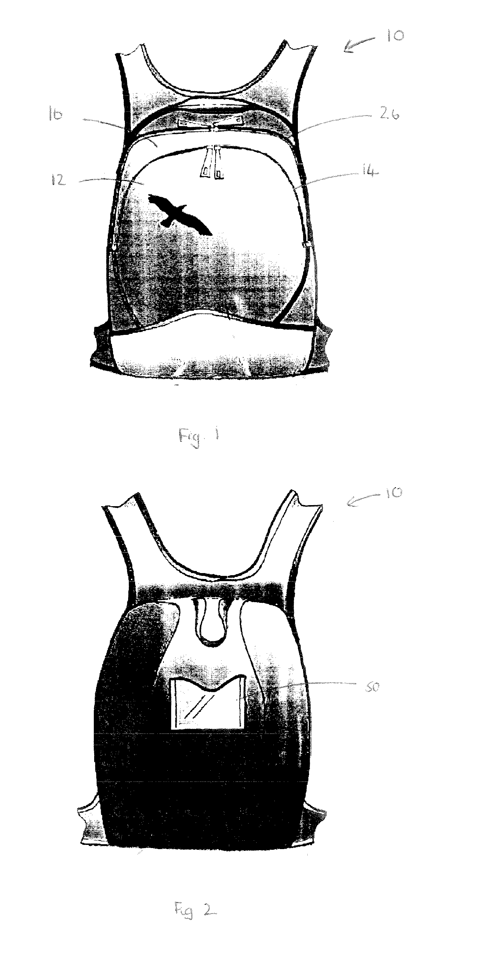

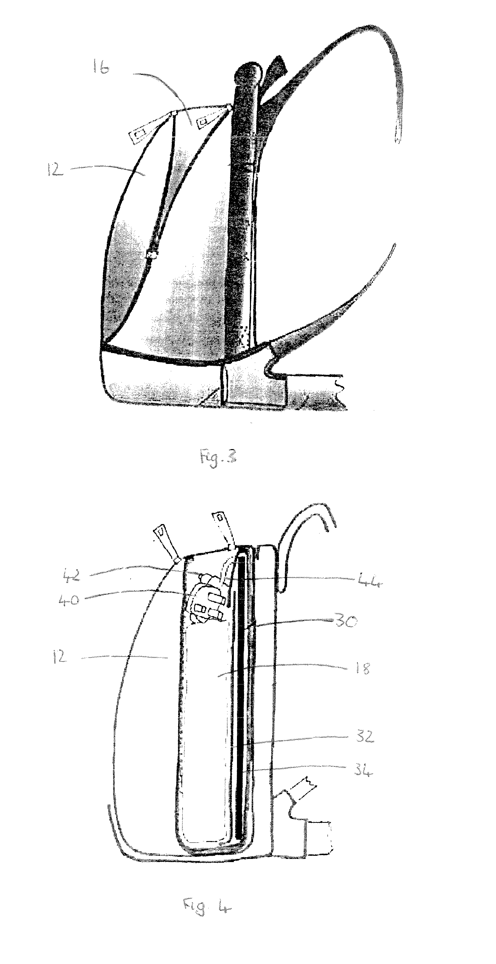

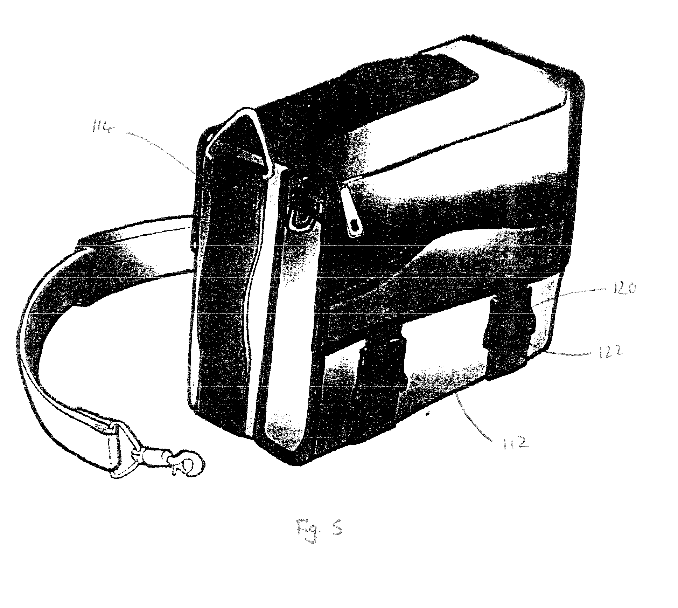

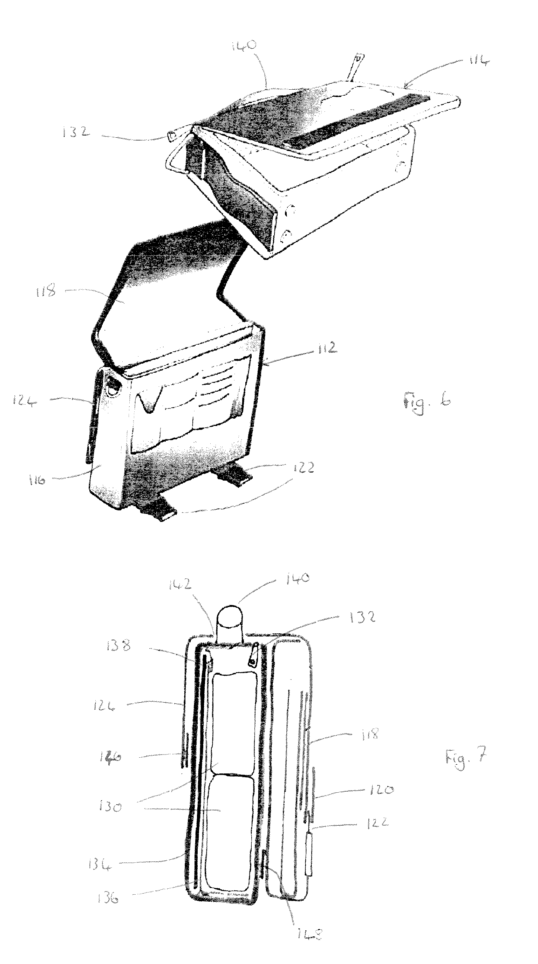

Figures 1-4 show a rucksack 10 according to a first embodiment of the present invention. The rucksack 10 is designed to look similar to conventional rucksacks and has a first compartment 12 for use as a conventional rucksack compartment. The first compartment 12 is accessed via a zip 14. The rucksack 10 also has a second compartment 16, which is designed to receive two dialysis bags 18 (only one shown in Figure 4). The second compartment 16 is accessed via a zip 20.

The second compartment 16 is sized to maintain the dialysis bags 18 in close contact with the rear wall 30. The rear wall 30 comprises a pocket 32 for receiving a heating mat 34 in the form of two heating elements 36, 38 sandwiched between two layers of rubber 46. The heating elements 36, 38 are shown in more detail in Figure 8. The mat 34 is sufficiently stiff that the heating elements will not break during normal use of the rucksack 10, whilst being flexible enough for comfort when being carried. However, the rucksack 10 may be provided with additional stiffening means such as metal rods, as is well known in rucksack design.

The heating elements 36, 38 are powered either by the mains or by a car battery. Figure 4 shows both a mains plug 40 and a cigarette lighter plug 42 together with respective cables 44 stored within the second compartment 16. The cables 44 are preferably permanently attached to their respective heating elements 36, 38.

Figure 8 shows an exemplary layout of a heating mat 34. The two heating elements 36, 38 are provided sandwiched between two layers of rubber 46 (only one shown for clarity) to form the mat 34. The mains powered heating element 36 at 230V extends over approximately 50% of the surface area of the mat 34 and the battery powered heating element 38 at 12V extends over the remaining 50% of the surface area of the mat 34.

The heating elements 36, 38 are preferably etch foil elements with a layer of silicone rubber 46 each side. The heating elements 36, 38 may be provided with a bimetal thermostat, which is adapted to maintain the temperature at or around body temperature. Preferably the thermostat cuts out at 50°C and resets at 35°C.

The walls of the second compartment 16 may be insulated to prevent the heat generated by the heating elements 36, 38 from escaping out of the second compartment 16. Many conventional insulation materials may be used, such as standard cool pack insulation. Foil may be placed behind the heating mat 34 to reflect heat back into the compartment 16. Silver backing may be provided on the inside of the compartment 16 to retain heat within the compartment 16.

The rear of the rucksack is provided with a card slot 50 for receiving a card which informs a person reading it that the bag contains dialysis equipment. However, the card is not visible when the bag is being carried.

In use, two dialysis bags 18 are placed in the second compartment 16. The rucksack 10 can be used as if it were a normal rucksack. When it is desired to heat the dialysis bags 18, the zip 20 of the second compartment 16 is undone to enable access to be gained to either the mains plug 40 or the cigarette lighter plug 42. The chosen plug 40, 42 and its respective cable 44 are passed through the opening of the second compartment 16 and the chosen plug 40, 42 is plugged in to an appropriate power source. When the dialysis bags 18 have reached a suitable temperature, preferably around body temperature, they may be removed from the rucksack 10 and used as is conventional in the art.

The rucksack 10 may be externally or internally provided with a light signal or audible alarm (not shown) to warn the user that the dialysis bags 18 are at a usable temperature. The light signal may be provided by coloured LEDs or heat sensitive strips that change colour with temperature.

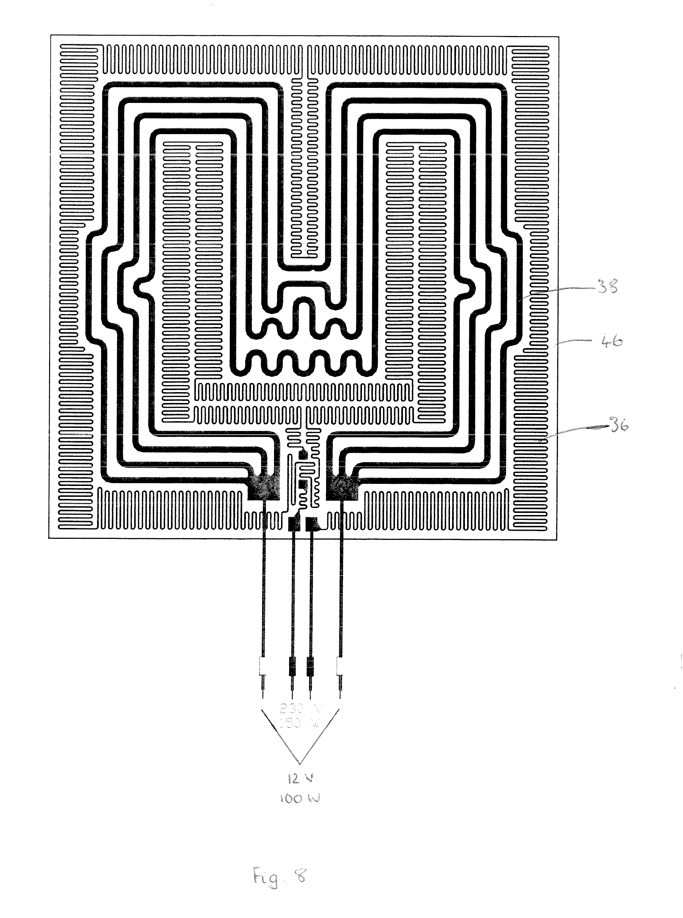

Figures 5 to 7 show a briefcase 110 according to a second embodiment of the invention. The briefcase 110 is designed to look similar to conventional briefcases. It comprises two sections 112 and 114, both of which can be used as separate cases in their own right or fastened together to form the single briefcase 110.

The first section 112 is designed for use as a conventional briefcase. The first section 112 comprises a main body 116 on which is mounted a closure flap 118 having two fastening means 120 thereon. Two corresponding fastening means 122 are located on a lower portion of the front face of the main body, each for receiving a fastening means 120. A second flap 124 extends over the rear of the briefcase and is held along the rear of the briefcase by means of Velcro^{™} or another quick release fitting. The second flap 124 has no function when the first section 112 is in use as a briefcase in its own right.

The second section 114 is designed to receive two dialysis bags 130, together with power supply connection means (not shown). The second section 114 accessed via a zip 132. The second section 114 is sized to maintain the dialysis bags 130 in close contact with the rear wall 134. The rear wall 134 comprises a rear pocket 136 in which a heating mat 138 is located. The heating mat 138 may be as set out in Figure 8 and as described in relation to Figures 1-4 above.

The briefcase 110 may be powered either by the mains or by a car battery. Both a mains plug and a cigarette lighter plug together with respective cables may stored within the second section 114.

The first and second sections 112, 114 may be held together to form a single briefcase by undoing the Velcro^{™} 126 between the rear flap 124 and the back wall 134 of the first section 112, passing a handle 140 of the second section 114 through a slot 142 in the rear flap 124 of the first section 112 and connecting the Velcro^{™} on the rear flap 124 to a different Velcro^{™} point 146 on the back wall 134 of the second section 114. Velcro^{™} 148 may also be located between the front surface of the second section 114 and the rear surface of the first section 112 to ensure a secure connection between the first sections 112 and the second section 114.

In use, two dialysis bags 130 are placed in the second section 114. The briefcase 110 can be used as if it were a normal briefcase, either with both the first section 112 and the second section 114 attached together, or with the just the second section alone. When it is desired to heat the dialysis bags 130, the zip 132 is undone to enable access to be gained to either the mains plug or the cigarette lighter plug. The chosen plug and cable are passed through the opening of the second section 114 and plugged in to an appropriate power source. The zip 132 is then closed to maintain the heat generated by the heating elements within the second section 114.

The briefcase 110 may be externally or internally provided with a light signal or audible alarm (not shown) to warn the user that the dialysis bags 130 are at a usable temperature. The light signal may be provided by coloured LEDs or heat sensitive strips that change colour with temperature.

The dialysis equipment bags of the present invention have the advantage that they are light and easy to carry. Furthermore, when the dialysis equipment bags are not in use to heat dialysis bags, they may show no visually obvious external signs of the fact that they are designed to carry and heat dialysis bags.

Although a few preferred embodiments have been shown and described, it will be appreciated by those skilled in the art that various changes and modifications might be made without departing from the scope of the invention, as defined in the appended claims.

Attention is directed to all papers and documents which are filed concurrently with or previous to this specification in connection with this application and which are open to public inspection with this specification, and the contents of all such papers and documents are incorporated herein by reference.

All of the features disclosed in this specification (including any accompanying claims, abstract and drawings), and/or all of the steps of any method or process so disclosed, may be combined in any combination, except combinations where at least some of such features and/or steps are mutually exclusive.

Each feature disclosed in this specification (including any accompanying claims, abstract and drawings) may be replaced by alternative features serving the same, equivalent or similar purpose, unless expressly stated otherwise. Thus, unless expressly stated otherwise, each feature disclosed is one example only of a generic series of equivalent or similar features.

The invention is not restricted to the details of the foregoing embodiment(s). The invention extends to any novel one, or any novel combination, of the features disclosed in this specification (including any accompanying claims, abstract and drawings), or to any novel one, or any novel combination, of the steps of any method or process so disclosed.

## Claims

1. A dialysis equipment bag, wherein the dialysis equipment bag is adapted to receive at least one dialysis bag; and the dialysis equipment bag comprises heating means for warming said at least one dialysis bag.

2. A dialysis equipment bag according to claim 1, wherein the dialysis equipment bag comprises a dialysis bag compartment adapted to receive the or each dialysis bag.

3. A dialysis equipment bag according to claim 2, wherein the dialysis equipment bag is provided with one or more additional storage compartments for carrying objects other than dialysis bags.

4. A dialysis equipment bag according to any preceding claim, wherein the heating means are electrical heating means.

5. A dialysis equipment bag according to claim 4, wherein the heating means are powered by the mains and/or operated by a battery.

6. A dialysis equipment bag according to claim 4 or 5, wherein the heating means comprises at least one heating element.

7. A dialysis equipment bag according to claim 6, wherein the at least one heating element comprises a first heating element which is connectable to the mains and a second heating element which is connectable to the car battery.

8. A dialysis equipment bag according to claim 6 or 7, wherein the at least one heating element may be provided with a bimetal thermostat.

9. A dialysis equipment bag according to any preceding claim, wherein the or each dialysis bag compartment is lined with heat storing material.

10. A dialysis equipment bag according to any preceding claim, wherein a light signal or audible alarm may be provided externally or internally of the dialysis equipment bag to warn the user that the dialysis bags are at a usable temperature.

11. A dialysis equipment bag according to any preceding claim, wherein the dialysis equipment bag is a rucksack or a briefcase.

12. A method of operating the dialysis equipment bag of any preceding claim.
